# DEMANDE DE BREVET EUROPEEN

(11) **EP 2 108 405 A1**
(43) Date de publication de la demande: **14.10.2009**
(21) Numéro de dépôt: 09305297.5
(22) Date de dépôt: 07.04.2009
(51) Int. Cl.: A61Q 5/04, A61K 8/19, A61K 8/20, A61K 8/36, A61K 8/43

(54) **Procede de deformation permanente des fibres keratiniques presentant une etape de pretraitement acide**

(30) Priorité: 08.04.2008 FR 0852355
(71) Demandeur: L'Oreal, 75008 Paris (FR)
(72) Inventeur: Barbarat, Philippe, 92270, Bois-Colombes (FR); Nuzzo, Stefania, 75014, Paris (FR)
(74) Mandataire: Catherine, Alain

(57) **Abrégé**

L'invention concerne un procédé de déformation permanente des fibres kératiniques, en particulier des cheveux, comprenant :
- une étape d'application sur les fibres kératiniques d'une composition de prétraitement comprenant dans un milieu cosmétiquement acceptable au moins un acide minéral et/ou organique non réducteur, et/ou l'un de ses sels, le pH de cette composition de prétraitement étant compris entre 2 et 5, préférentiellement entre 2,5 et 4 puis
- une étape de rinçage des fibres kératiniques, de préférence à l'eau, puis
- une étape d'application sur les fibres kératiniques d'une composition de traitement comprenant dans un milieu cosmétiquement acceptable au moins un composé hydroxyde,
- une étape de mise en forme des fibres kératiniques s'effectuant après l'application de la composition de prétraitement et avant, pendant ou après l'application de la composition de traitement.

## Description

La présente invention se rapporte à un procédé de déformation permanente des fibres kératiniques, en particulier de défrisage, comprenant une étape d'application d'une composition acide et une étape d'application d'une composition comprenant un composé hydroxyde.

Deux techniques sont généralement utilisées pour obtenir une déformation permanente des cheveux. Elles sont basées sur une rupture des liaisons disulfures -S-S- présentes dans la kératine (cystine).

La première technique pour obtenir une déformation permanente des cheveux consiste à réaliser dans un premier temps l'ouverture des liaisons disulfures à l'aide d'une composition contenant un agent réducteur (étape de réduction), puis après avoir, de préférence, rincé les cheveux, à reconstituer dans un second temps lesdites liaisons disulfures en appliquant sur les cheveux préalablement mis sous tension par des bigoudis ou autres ou mis en forme ou lissés par d'autres moyens, une composition oxydante encore appelée fixateur (étape d'oxydation), de façon à donner à la chevelure la forme recherchée. Cette technique permet indifféremment de réaliser, soit l'ondulation des cheveux, soit leur mise en forme ou leur décrêpage ou leur lissage.

La seconde technique pour obtenir une déformation permanente de cheveux consiste à effectuer une opération dite de lanthionisation, à l'aide d'une composition contenant une base appartenant à la famille des hydroxydes. Elle conduit à remplacer des liaisons disulfures (-CH₂-S-S-CH₂-) par des liaisons lanthionines (-CH₂-S-CH₂-). Cette opération de lanthionisation fait intervenir deux réactions chimiques consécutives :
■ La première réaction consiste en une béta-élimination sur la cystine provoquée par un ion hydroxyde, conduisant à la rupture de cette liaison et à la formation de déhydro-alanine, comme cela est représenté sur le schéma réactionnel suivant. 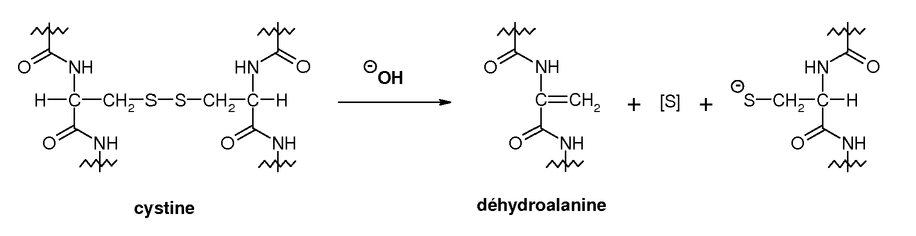
■ La deuxième réaction est une réaction de la déhydro-alanine avec un groupe thiol. En effet, la double-liaison de la déhydro-alanine formée est une double-liaison réactive. Elle peut réagir avec le groupe thiol du résidu cystéine qui a été libéré pour former une nouvelle liaison appelée pont ou liaison ou résidu lanthionine. Cette deuxième réaction est illustrée par le schéma réactionnel suivant.

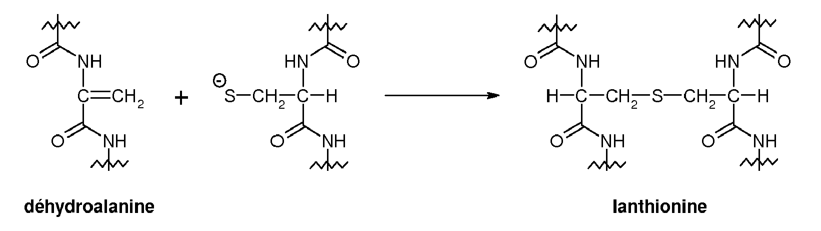

Par rapport à la première technique décrite précédemment mettant en oeuvre un agent réducteur, cette technique de lanthionisation ne nécessite pas d'étape de fixation, puisque la formation des ponts lanthionine est irréversible. Elle s'effectue donc en une seule étape et permet indifféremment de réaliser soit l'ondulation des cheveux, soit leur mise en forme ou leur décrêpage ou leur lissage. Cependant, elle est principalement utilisée pour la mise en forme des cheveux naturellement crépus.

D'une façon générale, la durabilité des effets de déformations obtenus avec les thiols et les sulfites par réduction des disulfures puis fixation est très inférieure à celle pouvant être obtenue par la technique de lanthionisation.

Les compositions généralement utilisées pour effectuer la lanthionisation contiennent à titre de base un hydroxyde tel que l'hydroxyde de sodium, l'hydroxyde de guanidinium et l'hydroxyde de lithium. L'hydroxyde de sodium et l'hydroxyde de guanidinium sont les deux agents principaux utilisés pour la mise en forme ou le décrêpage des cheveux naturellement crépus. Ils possèdent plusieurs avantages par rapport au thioglycolate d'ammonium et aux sulfites, en particulier une absence d'odeur désagréable, le fait qu'une seule étape de mise en oeuvre est requise pour la déformation des cheveux (et donc une durée du traitement plus courte), et ils conduisent de façon plus efficace à une déformation du cheveu qui présente une durabilité beaucoup plus importante.

Cependant, ces hydroxydes, utilisés sans prétraitement acide, présentent l'inconvénient majeur d'être caustiques.

Cette causticité affecte le cuir chevelu en provoquant des irritations parfois sévères. Il est connu qu'on peut y remédier partiellement par l'application préalable sur le cuir chevelu d'une crème protectrice grasse souvent appelée " base " ou " crème base ", le mot " base " ici utilisé n'ayant pas la signification d'agent basique au sens chimique. Lorsque la crème protectrice est associée à l'hydroxyde en une seule composition, celle-ci est généralement appelée " no-base ", par opposition à l'appellation ci-dessus. Cette technologie " no-base " est généralement préférée.

La causticité des hydroxydes affecte également l'état du cheveu en le rendant d'une part rêche au toucher et d'autre part beaucoup plus fragile, cette fragilité pouvant aller jusqu'à l'effritement voire la rupture ou même la dissolution des cheveux si le traitement est prolongé. Les hydroxydes provoquent dans certains cas également des décolorations de la couleur naturelle du cheveu.

De plus, les hydroxydes sont connus pour être de bons agents d'hydrolyse des fonctions amides (voir par exemple March's Advanced Organic Chemistry, 5ed., Wiley Interscience, New York, " Hydrolysis of Amides " pages 474 et suivantes) qui conduisent donc à la rupture des liaisons peptidiques par attaque nucléophile directe. Sans être lié par une théorie, Il est ainsi toutefois probable que les altérations constatées au niveau des cheveux et des matières kératiniques au sens large soient en grande partie dues à une hydrolyse partielle des liaisons amides de la kératine.

Ainsi, les traitements de défrisage connus altèrent fortement la qualité du cheveu. Ces altérations sont la conséquence de modifications importantes des propriétés physiques, chimiques et physico-chimiques intrinsèques du cheveu. Parmi ces propriétés figurent les propriétés de diffusion qui régissent le gonflement et la porosité du cheveu par exemple. Les traitements de défrisage alcalin (à la soude ou équivalent) sont connus pour altérer de façon importante les propriétés de diffusion du cheveu.

Il existe donc un réel besoin pour la mise en forme des cheveux de compositions moins agressives pour le cheveu.

Des traitements acides en post-traitements sont connus pour améliorer la qualité du cheveu après un traitement cosmétique tel que la décoloration, la permanente et le défrisage.

Par ailleurs, on connaît du document WO 01/06997 une composition anti-radicaux libres de pré-traitement capillaire à appliquer avant un traitement chimique, tel que le frisage ou le lissage des cheveux. La composition de pré-traitement contient de l'acide ascorbique. Mais il n'est pas fait état dans ce document d'une déformation permanente à la soude.

On connaît encore du document US 4 709 712 l'utilisation d'une composition sous forme de gel aqueux comprenant un polymère acide polycarboxylique pour protéger le cuir chevelu d'un écoulement d'une solution caustique pendant le traitement des cheveux, en particulier pendant une déformation permanente des cheveux. Mais il n'est pas fait mention d'une déformation permanente à la soude (lanthionisation).

On connaît également du document WO02/085317 un procédé de lanthionisation des fibres kératiniques comprenant l'application d'une composition de prétraitement comprenant au moins un nucléophile organique permettant d'accroître la charge de rupture des fibres kératiniques, puis l'application d'une composition de relaxation permettant la lanthionisation. Le nucléophile organique peut être par exemple un dérivé d'acide aminé basique.

On connaît également du document WO 03/015731 un procédé de lanthionisation des fibres kératiniques comprenant l'application d'une composition de prétraitement contenant au moins un agent réducteur et l'application d'une composition comprenant un générateur d'ions hydroxyde pour réaliser la lanthionisation.

L'invention vise donc à fournir un procédé de déformation permanente, en particulier un procédé de défrisage, des fibres kératiniques, qui améliore la qualité de la fibre, notamment la douceur, la brillance et le toucher, qui limite l'altération des propriétés de diffusion de la fibre, en particulier qui permet une diminution du gonflement dans l'eau de la fibre.

L'invention a donc pour objet un procédé de déformation permanente des fibres kératiniques, en particulier des cheveux, comprenant :
- une étape d'application sur les fibres kératiniques d'une composition de prétraitement comprenant dans un milieu cosmétiquement acceptable au moins un acide minéral et/ou organique non réducteur, et/ou l'un de ses sels, le pH de cette composition de prétraitement étant compris entre 2 et 5, préférentiellement entre 2,5 et 4 puis
- une étape de rinçage des fibres kératiniques, de préférence à l'eau, puis
- une étape d'application sur les fibres kératiniques d'une composition de traitement comprenant dans un milieu cosmétiquement acceptable au moins un composé hydroxyde,
- une étape de mise en forme des fibres kératiniques s'effectuant après l'application de la composition de prétraitement et avant, pendant ou après l'application de la composition de traitement.

Le procédé selon l'invention comprend donc d'abord une étape d'application d'une composition de prétraitement.

Comme expliqué précédemment, la composition de prétraitement comprend un ou plusieurs acides minéraux et/ou organiques non réducteurs, et/ou un de leurs sels.

Le ou les acides minéraux non réducteurs présents dans la composition de prétraitement peuvent être choisis parmi les monoacides et les polyacides.

Généralement, le ou les acides minéraux non réducteurs sont choisis parmi l'acide chlorhydrique, l'acide phosphorique, l'acide sulfurique et l'acide borique.

Le ou les acides organiques non réducteurs présents dans la composition de prétraitement sont généralement choisis parmi les acides comportant une ou plusieurs fonctions acide carboxylique, sulfonique, phosphonique ou phosphorique. Ils peuvent contenir d'autres fonctions chimiques en particulier des fonctions hydroxy ou amino. Ils peuvent être saturés ou insaturés.

Les acides monocarboxyliques non réducteurs sont généralement choisis parmi les acides aliphatiques ou aromatiques, hydroxylés ou non. On peut citer en particulier l'acide acétique, l'acide trichloracétique, l'acide propanoïque, l'acide butanoïque, l'acide phényl acétique, l'acide salicylique, l'acide benzylique, l'acide glycolique, l'acide lactique, l'acide ascorbique, les dérivés d'acide salicylique en particulier l'acide 2-hydroxy-5-octanoyl-benzoïque et l'acide 2-hydroxy-4-triméthylsilanylméthoxy benzoïque, le dérivé d'acide jasmonique, l'acide 3-hydroxy-2-pentyl-cyclopentyl acétique, l'acide pyruvique et l'acide mandélique.

Les acides polycarboxyliques non réducteurs sont généralement choisis parmi l'acide aspartique, l'acide glutamique, l'acide oxalique, l'acide succinique, l'acide tartrique, l'acide mucique, l'acide citrique, l'acide malique, l'acide maléique, l'acide phtalique, les éthers poly(éthylène glycol) bis(carboxyméthyl), le polyacide acrylique, le copolymère acide acrylique/maléique, l'acide polyaspartique, les polydiméthylsiloxanes carboxyliques.

Le polyacide acrylique non réducteur utilisé a généralement une masse moléculaire de 2000 g/mol. Le copolymère acide acrylique/maléique utilisé a généralement une masse moléculaire de 3000 g/mol. L'acide polyaspartique utilisé a généralement une masse moléculaire de 3000 g/mol. Les polydiméthylsiloxane carboxyliques utilisés ont généralement une masse moléculaire de 920 g/mol ou bien de 2330 g/mol. Les éthers poly(éthylène glycol) bis(carboxyméthyl) utilisés ont généralement une masse moléculaire de 250 g/mol ou bien de 600 g/mol.

Les acides sulfoniques non réducteurs peuvent être choisis parmi l'acide benzène sulfonique, l'acide sulfonique HSO₂OH, la taurine (acide 2-aminoethane sulfonique), l'acide 2-[4-(2-hydroxy-éthyl)-pipérazin-1-yl]éthanesulfonique (ou HEPES) et le (3E)-3-(4-{(E)-[7,7-diméthyl-3-oxo-4-(sulfométhyl)bicyclo[2.2.1 ]hept-2-ylidène]méthyl}benzylidène)-7,7-diméthyl-2-oxobicyclo[2.2.1]hept-1-yl]méthanesulfonique, ce dernier composé étant commercialisé par la société Chimex sous la référence MEXORYL SX. La formule chimique du MEXORYL SX est la suivante :

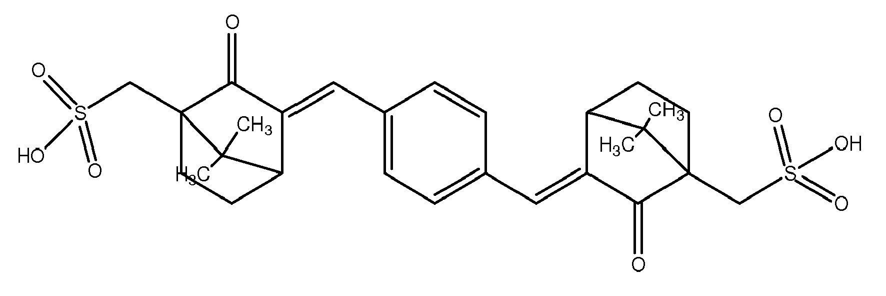

Le ou les acides organiques non réducteurs utilisés de façon préférée dans la composition de prétraitement sont des acides faibles, c'est-à-dire des acides qui ne se dissocient pas totalement dans l'eau et dont le pKa est supérieur à 0. Parmi ces acides faibles, on utilise de préférence l'acide citrique, l'acide acétique, l'acide salicylique, l'acide glycolique, l'acide trichloracétique, l'acide L-glutamique, l'acide lactique, l'acide succinique, l'acide DL tartrique, l'éther poly(éthylène glycol) bis(carboxyméthyl) de masse moléculaire de 250 g/mol, les dérivés d'acide salicylique en particulier l'acide 2-hydroxy-5-octanoyl-benzoïque et l'acide 2-hydroxy-4-triméthylsilanylméthoxy-benzoïque, le dérivé d'acide jasmonique, l'acide 3-hydroxy-2-pentyl-cyclopentyl acétique, l'acide 2-[4-(2-hydroxy-éthyl)-piperazin-1-yl]-éthanesulfonique, l'acide malique, l'acide pyruvique et l'acide mandélique.

Les acides organiques non réducteurs utilisés de façon particulièrement préférée dans la composition de prétraitement sont l'acide citrique, l'acide acétique, l'acide salicylique et l'acide glycolique.

La composition de prétraitement peut en outre renfermer plusieurs acides et/ou leurs sels tels que définis précédemment.

Le pH de la composition de prétraitement est compris entre 2 et 5, mieux entre 2,5 et 4.

Pour cela, le pH est généralement stabilisé à l'aide d'une solution tampon.

Par solution tampon, on entend une solution dont le pH ne varie pas par dilution ou par ajout d'une faible quantité de base ou d'acide.

La formulation des solutions tampons est bien connue de l'homme du métier.

Lorsque la composition de prétraitement est appliquée sur les fibres kératiniques, sa température est avantageusement comprise entre 25 et 60°C, de préférence entre 35 et 50°C.

Généralement, on laisse la composition de prétraitement agir pendant 10 à 60 minutes, de préférence pendant 10 à 40 minutes.

Après l'application de la composition de prétraitement et le temps de pose éventuel, les fibres kératiniques sont rincées, de préférence à l'eau.

Après l'étape de rinçage, et avant l'application de la composition de traitement, les fibres kératiniques sont de préférence essorées, par exemple entre deux serviettes. Il est préférable d'éviter de sécher totalement les fibres kératiniques.

Comme expliqué précédemment, le procédé selon l'invention comprend, après l'étape d'application de la composition de prétraitement, une étape d'application sur les fibres kératiniques d'une composition de traitement comprenant dans un milieu cosmétiquement acceptable au moins un composé hydroxyde.

Par composé hydroxyde, on entend au sens de la présente invention un composé susceptible de libérer des ions hydroxydes.

Tous les composés hydroxydes habituellement utilisés dans les procédés de lanthionisation peuvent être utilisés dans la composition de traitement utilisée selon l'invention.

Le ou les composés hydroxydes présents dans la composition de traitement utilisée selon l'invention sont de préférence choisis parmi les hydroxydes de métaux alcalins, les hydroxydes de métaux alcalino-terreux, les hydroxydes de métaux de transition, les hydroxydes de métaux lanthanides, les hydroxydes de métaux actinides, les hydroxydes du Groupe III, les hydroxydes du Groupe IV, les hydroxydes du Groupe V, les hydroxydes du Groupe VI, les hydroxydes organiques, et les composés comprenant au moins un substituant hydroxyde partiellement hydrolysable.

A titre de composés hydroxydes utilisables selon l'invention, on peut citer en particulier l'hydroxyde de sodium, l'hydroxyde de guanidinium, l'hydroxyde de lithium, l'hydroxyde de calcium, l'hydroxyde de barium, l'hydroxyde de potassium, l'hydroxyde de magnésium, l'hydroxyde d'aluminium, l'hydroxyde de cuivre et l'hydroxyde de zinc.

Les composés hydroxydes préférés sont l'hydroxyde de sodium et l'hydroxyde de guanidinium.

La composition de traitement utilisée dans le procédé selon l'invention se présente généralement sous la forme d'une émulsion, de préférence huile-dans-eau ou eau-dans-huile.

Lorsque la composition de traitement utilisée selon l'invention est sous forme d'émulsion, elle contient généralement au moins un agent émulsionnant non-ionique, anionique, cationique ou amphotère.

Les émulsionnants sont choisis de manière appropriée suivant l'émulsion à obtenir : émulsion eau-dans-l'huile (E/H) ou huile-dans-eau (H/E).

Pour les émulsions huile-dans-eau (H/E), on peut citer par exemple les émulsionnants suivants:
* comme émulsionnants amphotères, les N-acyl-aminoacides tels que les N-alkylaminoacétates et le cocoamphodiacétate disodique et les oxydes d'amines tels que l'oxyde de stéaramine;
* comme émulsionnants anioniques, les acylglutamates tels que le "disodium hydrogenated tallow glutamate" (Amisoft HS-21® commercialisé par la société AJINOMOTO) ; les acides carboxyliques et leurs sels tels que le stéarate de sodium ; les esters phosphoriques et leurs sels tels que le "DEA oleth-10 phosphate" ; les sulfosuccinates tels que le "Disodium PEG-5 citrate lauryl sulfosuccinate" et le "Disodium ricinoleamido MEA sulfosuccinate" ;
* comme émulsionnants cationiques, les alkyl-imidazolidinium tels que l'éthosulfate d'isostéaryl-éthylimidonium ; les sels d'ammonium tels que le chlorure de N,N,N-triméthyl-1-docosanaminium (Behentrimonium chloride);
* comme émulsionnants non ioniques, les esters et éthers d'oses tels que le stéarate de sucrose, le cocoate de sucrose, et le mélange de stéarate de sorbitan et de cocoate de sucrose commercialisé par la société ICI sous la dénomination Arlatone 2121® ; les esters de polyol, notamment de glycérol ou de sorbitol, tels que le stéarate de glycéryle, le stéarate de polyglycéryl-2, le stéarate de sorbitan ; les éthers de glycérol ; les éthers oxyéthylénés et/ou oxypropylénés, tels que l'éther oxyéthyléné, oxypropyléné de l'alcool laurique à 25 groupes oxyéthylénés et 25 groupes oxypropylénés (nom CTFA "PPG-25 laureth-25") et l'éther oxyéthyléné du mélange d'alcools gras en C₁₂-C₁₅ comportant 7 groupes oxyéthylénés (nom CTFA " C₁₂-C₁₅ Pareth-7") ; les polymères d'éthylène glycol, tels que le PEG-1 00, et leurs mélanges.

On peut utiliser un ou plusieurs de ces émulsionnants.

Pour les émulsions eau-dans-huile (E/H), on peut citer comme exemple d'émulsionnants, les esters gras de polyol, notamment de glycérol ou de sorbitol, et notamment les esters isostéariques, oléiques et ricinoléiques de polyol, tels que le mélange de petrolatum, d'oléate de polyglycéryl-3, d'isostéarate de glycéryle, l'huile de ricin hydrogénée et d'ozokérite, vendu sous la dénomination Protegin W® par la société GOLDSCHMIDT, l'isostéarate de sorbitan, le di-isostéarate de polyglycéryle, le sesqui-isostéarate de polyglycéryle-2 ; les esters et éthers d'oses tels que le "Methyl glucose dioléate" ; les esters gras tels que le lanolate de magnésium ; les diméthicone copolyols et alkyl-diméthicone copolyols tels que le laurylméthicone copolyol vendu sous la dénomination Dow Corning 5200 Formulation Aid par la société DOW CORNING, le Cétyl diméthicone copolyol vendu sous la dénomination Abil EM 90® par la société GOLDSCHMIDT, et le diméthicone copolyol vendu sous la dénomination KF-6015 par la société SHIN-ETSU ; et leurs mélanges.

Le ou les composés hydroxydes sont généralement présents dans la composition de traitement à une concentration comprise entre 0,2 et 1 M, de préférence entre 0,4 et 0,6M.

Lorsque la composition de traitement est appliquée sur les fibres kératiniques, sa température est de préférence comprise entre 20 et 40°C, mieux entre 25 et 35°C.

La composition de traitement est généralement laissée agir pendant un temps suffisant pour que la déformation des cheveux se fasse. En général, on laisse agir la composition de traitement pendant 5 à 60 minutes, de préférence pendant 10 à 20 minutes.

La lanthionisation est terminée lorsque le niveau souhaité de déformation des cheveux est atteint.

Après l'étape d'application de la composition de traitement, et après l'éventuel temps de pause, les fibres kératiniques sont généralement rincées, de préférence à l'eau.

Selon un mode de réalisation préféré de l'invention, le procédé selon l'invention comprend une étape d'application sur les fibres kératiniques d'une composition de post-traitement comprenant au moins un acide minéral et/ou organique, et/ou un de ses sels.

Le ou les acides organiques présents dans la composition de post-traitement sont généralement choisis parmi les acides comportant une ou plusieurs fonctions acide carboxylique, sulfonique, phosphonique ou phosphorique. Ils peuvent contenir d'autres fonctions chimiques en particulier des fonctions hydroxy ou amino. Ils peuvent être saturés ou insaturés.

On peut citer en particulier l'acide acétique, l'acide lactique, l'acide glycolique, l'acide ascorbique, l'acide malique, l'acide benzylique, l'acide succinique, la taurine, l'acide tartrique et l'acide citrique.

Un acide préféré est l'acide citrique.

Le ou les acides minéraux présents dans la composition de post-traitement sont généralement choisis parmi les monoacides ou polyacides.

On peut citer en particulier l'acide chlorhydrique, l'acide orthophosphorique, l'acide sulfurique et l'acide borique.

La composition de post-traitement présente généralement un pH compris entre 2 et 7, de préférence entre 2,5 et 4. Le pH de la composition peut être ajusté à l'aide d'un agent alcalin choisi parmi l'ammoniaque, la monoéthanolamine, la diéthanolamine, la triéthanolamine, la propanediamine-1,3, le carbonate ou bicarbonate d'ammonium, un carbonate organique ou un hydroxyde alcalin. La composition est généralement appliquée à température ambiante pendant 1 à 20 minutes, de préférence 3 à 10 minutes.

Comme expliqué précédemment, le ou les acides non réducteurs, minéraux et/ou organiques présents dans la composition de pré-traitement, le ou les composés hydroxydes présents dans la composition de traitement et le ou les acides organiques et/ou minéraux présents dans la composition de post-traitement sont présents dans un milieu cosmétiquement acceptable.

Le milieu cosmétiquement acceptable de la composition de pré-traitement, celui de la composition de traitement et celui de la composition de post-traitement sont généralement choisis indépendamment parmi l'eau, les alcools en C₁-C₆, de préférence les alcanols tels que l'éthanol, le propanol et l'isopropanol, les alcanediols tels que l'éthylèneglycol, le propylène glycol et le pentanediol, l'alcool benzylique, les alcanes en C₅-C₁₀, l'acétone, la méthyléthylcétone, les esters en C₂-C₆, tels que l'acétate de méthyle, l'acétate de butyle et l'acétate d'éthyle, le diméthoxyéthane, le diéthoxyéthane, la N-méthylpyrrolidone (NMP), le diméthylsulfoxyde (DMSO) et leurs mélanges.

La composition de pré-traitement, la composition de traitement et la composition de post-traitement peuvent en outre comprendre des additifs cosmétiques usuels choisis notamment parmi les agents épaississants, les adoucissants, les agents anti-mousse, les filtres solaires, les agents hydratants, les colorants, les pigments, les parfums, les conservateurs, les tensioactifs anioniques, cationiques, non ioniques ou amphotères, les polymères non fixants, les silicones volatiles ou non, les huiles végétales, animales, minérales ou de synthèse, les protéines, les vitamines, les polyols et leurs mélanges.

Selon un mode de réalisation particulier de l'invention, les fibres kératiniques sont soumises à un traitement thermique au moyen d'un fer chauffant à une température comprise entre 60 et 220°C, de préférence entre 120 et 220°C, cette étape de traitement thermique se faisant pendant ou après l'application de la composition de traitement.

L'invention a encore pour objet un kit cosmétique pour la déformation permanente des cheveux comprenant dans un premier compartiment une composition de prétraitement telle que définie précédemment, et dans un second compartiment une composition de traitement telle que définie précédemment. Le cas échéant, le kit peut comprendre un troisième compartiment renfermant la composition de post-traitement.

L'invention est illustrée par les exemples suivants.

### Exemples

### Exemple 1

### 1. Des cheveux naturels africains crépus sont défrisés en utilisant le procédé selon l'invention.

La composition de prétraitement utilisée est une solution tampon Titrisol pH3 contenant 0,04 mol/L d'acide citrique, 0,06 mol/L d'acide chlorhydrique et 0,08 mol/L de soude.

Les cheveux sont plongés pendant 30 minutes dans un bain à 40°C de la composition de prétraitement, avec un rapport de bain de 10 mL de composition de prétraitement par gramme de cheveux.

Les cheveux sont ensuite rincés à l'eau.

Les cheveux sont essorés entre 2 chiffons.

Puis les cheveux subissent une étape de défrisage.

Le défrisage des cheveux est effectué au moyen d'une composition de traitement de soude 0,6M (de pH 14 avant le traitement) pendant 20 minutes à 30°C. Cette étape présente les caractéristiques suivantes.

Les cheveux sont plongés dans la composition de soude. Le rapport de bain est de 40 ml/g, soit 10 ml par méchette.

Les cheveux sont étirés pendant 4 minutes, puis laissés non étirés pendant 6 minutes.

La mèche est alors retournée, et l'opération est renouvelée : 4 minutes d'étirement et 6 minutes de non étirement.

Les cheveux sont alors rincés par immersion dans de l'eau.

Les cheveux sont ensuite essorés entre deux chiffons.

Les cheveux sont plongés dans un bain d'acide citrique à pH 3 pendant 5 minutes à température ambiante. Le rapport de bain est environ de 50 ml/g.

Les cheveux sont ensuite rincés à l'eau et lavés au moyen d'un shampooing avant de sécher à l'air ambiant.

### 2. On étudie l'influence de l'étape d'application de la composition de pré-traitement sur les propriétés de mise en forme.

La performance de défrisage a été étudiée qualitativement.

On a comparé la mise en forme des cheveux traités par le procédé selon l'invention décrit au point 1, avec des cheveux témoins n'ayant pas fait l'objet d'une mise en forme, et avec des cheveux ayant fait l'objet d'une mise en forme selon le procédé décrit au point 1 mais dans lequel l'étape d'application d'une composition de prétraitement n'a pas été effectuée.

Les observations montrent que le prétraitement au moyen d'une composition de prétraitement contenant un acide ne perturbe pas la performance de mise en forme d'un procédé de défrisage à la soude.

### Exemple 2

### 1. On étudie l'amélioration de la qualité du cheveu apportée par le procédé selon l'invention. En particulier, on étudie le gonflement du cheveu dans l'eau.

Tous les traitements sont effectués sur des méchettes de 250 mg de cheveux africains.

Un premier échantillon (échantillon n°1) de cheveux est mis en forme en utilisant le procédé selon l'invention. Ainsi, les cheveux subissent les étapes suivantes :
- application sur les cheveux d'une composition de prétraitement d'acide acétique, de pH 3, à 40°C pendant 30 minutes ; le rapport de bain est de 10 ml/g ;
- rinçage à l'eau courante ;
- essorage des cheveux entre 2 chiffons ;
- défrisage des cheveux.

Le défrisage des cheveux est effectué au moyen d'une composition de traitement de soude 0,6M (de pH 14 avant le traitement) pendant 20 minutes à 30°C. Cette étape présente les caractéristiques suivantes.

Les cheveux sont plongés dans la composition de soude. Le rapport de bain est de 40 ml/g, soit 10 ml par méchette.

Les cheveux sont étirés pendant 4 minutes, puis laissés non étirés pendant 6 minutes.

La mèche est alors retournée, et l'opération est renouvelée : 4 minutes d'étirement et 6 minutes de non étirement.

Les cheveux sont alors rincés par immersion dans de l'eau.

Les cheveux sont ensuite essorés entre deux chiffons.

Les cheveux sont plongés dans un bain d'acide citrique à pH 3 pendant 5 minutes à température ambiante. Le rapport de bain est environ de 50 ml/g.

Les cheveux sont ensuite rincés à l'eau et lavés au moyen d'un shampooing avant de sécher à l'air ambiant.

Un autre échantillon (échantillon témoin) est défrisé au moyen de la composition de traitement définie ci-dessus, mais sans étape d'application d'une composition de prétraitement acide (ni donc de rinçage et d'essorage).

Un autre échantillon (échantillon n°2) est traité selon un procédé comprenant une étape de prétraitement, d'essorage et de défrisage telles que définies ci-dessus, mais pas d'étape de rinçage.

Un autre échantillon (échantillon n°3) est traité selon un procédé comprenant une étape de prétraitement telle que définie ci-dessus, une étape de rinçage telle que définie ci-dessus, une étape de séchage au casque à 60°C pendant 10 minutes, et une étape de défrisage à la soude telle que définie précédemment.

### 2. Les mesures de gonflement ont été réalisées à l'aide d'un système optique Zimmer 3D.

Le cheveu à analyser est introduit lesté dans une cuve de quartz remplie d'eau distillée à 25°C.

Le diamètre moyen du cheveu est évalué par la moyenne de 250 mesures contiguës à sec et dans l'eau. Cette étape de mesure dure environ 10 minutes.

Le gonflement moyen, qui traduit la qualité du cheveu, est déterminé par l'évolution du diamètre moyen entre l'état sec et l'état immergé.

### 3. Les résultats sont les suivants.

Les différents traitements conduisent à un niveau de défrisage équivalent.

La différence de qualité des cheveux après ces différents traitements a été évaluée par le test de gonflement à l'eau.

Les résultats obtenus sont présentés dans le tableau 1.

**Tableau 1**

| Echantillon | Traitement | Diamètre moyen sec (µm) | Diamètre moyen eau (µm) | Gonflement moyen | |
|---|---|---|---|---|---|
| | | | | Moyenne (%) | Ecart type (%) |
| Témoin | Pas de prétraitement | 91,3 | 115,9 | 27,4 | 9,0 |
| 1 | Prétraitement+rinçage + essorage+défrisage | 108,7 | 112,9 | 4,0 | 2,5 |
| 2 | Prétraitement+ essorage + défrisage | 76,3 | 96,4 | 20,9 | 4,7 |
| 3 | Prétraitement+rinçage + séchage+défrisage | 85,9 | 107,3 | 24,9 | 2,4 |

L'étape de rinçage intermédiaire a un effet positif sur la qualité du cheveu après défrisage, traduit par une diminution du gonflement du cheveu dans l'eau. Cet effet est significatif dès lors que cette étape de rinçage est suivie d'un essorage des cheveux, et non d'un séchage au casque ou au séchoir, avant l'étape de défrisage.

### Exemple 3

### A. On étudie l'amélioration de la qualité du cheveu apportée par le procédé selon l'invention. En particulier, on étudie le gonflement du cheveu dans l'eau.

Tous les traitements sont effectués sur des méchettes de 250 mg de cheveux africains.

Deux échantillons de cheveux sont mis en forme en utilisant le procédé selon l'invention. Ainsi, les cheveux subissent les étapes décrites ci-dessous.

### 1. Prétraitement acide

On applique sur les cheveux une composition de prétraitement, soit à base d'acide glycolique, soit à base d'acide salicylique, soit à base d'acide citrique.

La composition de prétraitement à base d'acide glycolique comprend 23 mg d'acide glycolique pour 10 ml d'eau.

La composition de prétraitement à base d'acide salicylique comprend 20 mg d'acide salicylique pour 10 ml d'eau.

La composition de prétraitement à base d'acide citrique comprend 2,7 mg d'acide citrique pour 10 ml d'eau.

La composition de prétraitement est appliquée sur les cheveux pendant 30 minutes à 40°C en bain-marie dans un bécher. Le rapport de bain est de 10 ml/g, soit 2,5 ml de composition par méchette.

### 2. Rinçage

Les cheveux sont rinçés à l'eau.

### 3. Essorage.

Les cheveux sont essorés entre 2 serviettes.

### 4. Défrisage

Le défrisage des cheveux est effectué au moyen d'une composition de traitement de soude 0,6M (de pH 14 avant le traitement) pendant 20 minutes à 30°C. Cette étape présente les caractéristiques suivantes.

Les cheveux sont plongés dans la composition de soude. Le rapport de bain est de 40 ml/g, soit 10 ml par méchette.

Les cheveux sont étirés pendant 4 minutes, puis laissés non étirés pendant 6 minutes.

La mèche est alors retournée, et l'opération est renouvelée : 4 minutes d'étirement et 6 minutes de non étirement.

Les cheveux sont alors rincés par immersion dans de l'eau.

Les cheveux sont ensuite essorés entre deux serviettes.

### 5. Neutralisation à l'acide citrique

Les cheveux sont plongés dans un bain d'acide citrique à pH 3 pendant 5 minutes à température ambiante. Le rapport de bain est environ de 50 ml/g. Les cheveux sont ensuite rincés à l'eau et lavés au moyen d'un shampooing avant de sécher à l'air ambiant.

### B. On évalue le gonflement dans l'eau du cheveu traité.

Les mesures de gonflement ont été réalisées à l'aide d'un système optique Zimmer permettant de suivre l'évolution du diamètre du cheveu lors de l'immersion dans l'eau. Le cheveu lesté est introduit dans une cuve de quartz remplie d'eau distillée à 25°C. L'évolution du diamètre est ensuite enregistrée en continu durant 5 minutes. A partir de 5 minutes, la valeur du diamètre est stabilisée.

A partir de ces mesures, on détermine le gonflement moyen de la fibre pour chaque traitement.

Les deux procédures de mesures de gonflement à l'aide du Zimmer 3D (exemple 2) et Zimmer (exemple 3) ne sont pas identiques, mais l'information obtenue est équivalente. Dans le cas du ZIMMER 3D, on attend quelques minutes et on mesure le diamètre du cheveu après stabilisation. On donne la valeur du diamètre stabilisée. Dans le cas du ZIMMER, on mesure l'évolution du diamètre du cheveu de 0 à 5 minutes, à partir de 5 minutes, on donne la valeur stabilisée du diamètre.

Les résultats sont issus de la moyenne de 15 essais pour chaque traitement.

Les cheveux témoins ayant été traité selon le procédé décrit ci-dessus à l'exclusion de l'étape de prétraitement acide présentent un gonflement dans l'eau de 50,43%.

Les cheveux ayant été traités selon le procédé décrit ci-dessus et dont le prétraitement acide a été effectué soit à l'aide de la composition d'acide citrique ou d'acide glycolique présentent un gonflement dans l'eau beaucoup plus faible que des cheveux n'ayant pas subi de prétraitement.

Le procédé selon l'invention a donc un effet positif sur la qualité du cheveu après défrisage, traduit par une diminution du gonflement du cheveu dans l'eau.

## Revendications

1. Procédé de déformation permanente des fibres kératiniques, en particulier des cheveux, comprenant :
- une étape d'application sur les fibres kératiniques d'une composition de prétraitement comprenant dans un milieu cosmétiquement acceptable au moins un acide minéral et/ou organique non réducteur, et/ou l'un de ses sels, le pH de cette composition de prétraitement étant compris entre 2 et 5, préférentiellement entre 2,5 et 4 puis
- une étape de rinçage des fibres kératiniques, de préférence à l'eau, puis
- une étape d'application sur les fibres kératiniques d'une composition de traitement comprenant dans un milieu cosmétiquement acceptable au moins un composé hydroxyde,
- une étape de mise en forme des fibres kératiniques s'effectuant après l'application de la composition de prétraitement et avant, pendant ou après l'application de la composition de traitement.

2. Procédé selon la revendication 1 **caractérisé en ce que** le ou les acides minéraux non réducteurs sont choisis parmi l'acide chlorhydrique, l'acide phosphorique, l'acide sulfurique et l'acide borique.

3. Procédé selon la revendication 1 **caractérisé en ce que** le ou les acides organiques non réducteurs sont des acides organiques monocarboxyliques choisis parmi l'acide acétique, l'acide trichloracétique, l'acide propanoïque, l'acide butanoïque, l'acide phényl acétique, l'acide salicylique, l'acide benzylique, l'acide glycolique, l'acide lactique, l'acide ascorbique, les dérivés d'acide salicylique en particulier l'acide 2-hydroxy-5-octanoyl-benzoïque et l'acide 2-hydroxy-4-triméthylsilanylméthoxy benzoïque, le dérivé d'acide jasmonique, l'acide 3-hydroxy-2-pentylcyclopentyl acétique, l'acide pyruvique et l'acide mandélique.

4. Procédé selon la revendication 1 **caractérisé en ce que** le ou les acides organiques non réducteurs sont des acides organiques polycarboxyliques choisis parmi l'acide aspartique, l'acide glutamique, l'acide oxalique, l'acide succinique, l'acide tartrique, l'acide mucique, l'acide citrique, l'acide malique, l'acide maléique, l'acide phtalique, les éthers poly(éthylène glycol) bis(carboxyméthyl), le polyacide acrylique, le copolymère acide acrylique/maléique, l'acide polyaspartique, les polydiméthylsiloxanes carboxyliques.

5. Procédé selon la revendication 1 **caractérisé en ce que** les acides organiques non réducteurs sont des acides sulfoniques choisis parmi l'acide benzène sulfonique, l'acide sulfonique HSO₂OH, la taurine, l'acide 2-[4-(2-hydroxy-éthyl)-pipérazin-1-yl] éthanesulfonique (ou HEPES) et le (3E)-3-(4-{(E)-[7,7-diméthyl-3-oxo-4-(sulfométhyl)bicyclo[2.2.1]hept-2-ylidène]méthyl}benzylidène)-7,7-diméthyl-2-oxobicyclo[2.2.1]hept-1-yl]méthanesulfonique,

6. Procédé selon les revendications 3 ou 4 **caractérisé en ce que** le ou les acides organiques non réducteurs sont choisis parmi l'acide citrique, l'acide acétique, l'acide salicylique et l'acide glycolique.

7. Procédé selon l'une quelconque des revendications **caractérisé en ce que**, après l'étape de rinçage, et avant l'étape d'application de la composition de traitement, les fibres kératiniques sont essorées.

8. Procédé selon l'une quelconque des revendications **caractérisé en ce que** le ou les composés hydroxydes sont choisis parmi les hydroxydes de métaux alcalins, les hydroxydes de métaux alcalino-terreux, les hydroxydes de métaux de transition, les hydroxydes de métaux lanthanides, les hydroxydes de métaux actinides, les hydroxydes du Groupe III, les hydroxydes du Groupe IV, les hydroxydes du Groupe V, les hydroxydes du Groupe VI, les hydroxydes organiques, et les composés comprenant au moins un substituant hydroxyde partiellement hydrolysable.

9. Procédé selon la revendication 8 **caractérisé en ce que** le ou les composés hydroxydes sont choisis parmi l'hydroxyde de sodium, l'hydroxyde de guanidinium, l'hydroxyde de lithium, l'hydroxyde de calcium, l'hydroxyde de barium, l'hydroxyde de potassium, l'hydroxyde de magnésium, l'hydroxyde d'aluminium, l'hydroxyde de cuivre et l'hydroxyde de zinc.

10. Procédé selon l'une quelconque des revendications précédentes **caractérisé en ce que** la composition de traitement est sous forme d'une émulsion, de préférence huile-dans-eau ou eau-dans-huile.

11. Procédé selon l'une quelconque des revendications précédentes **caractérisé en ce que** le ou les composés hydroxydes sont présents dans la composition de traitement à une concentration comprise entre 0,2 et 1 M, de préférence entre 0,4 et 0,6M.

12. Procédé selon l'une quelconque des revendications précédentes **caractérisé en ce qu'**il comprend une étape d'application sur les fibres kératiniques d'une composition de post-traitement comprenant dans un milieu cosmétiquement acceptable au moins un acide minéral et/ou organique et/ou un de ses sels.

13. Procédé selon la revendication 12 **caractérisé en ce que** le ou les acides présents dans la composition de post-traitement sont des acides organiques choisis parmi l'acide acétique, l'acide lactique, l'acide glycolique, l'acide ascorbique, l'acide malique, l'acide benzylique, l'acide succinique, la taurine, l'acide tartrique et l'acide citrique.

14. Procédé selon la revendication 12 **caractérisé en ce que** le ou les acides sont des acides minéraux choisis parmi l'acide chlorhydrique, l'acide phosphorique, l'acide sulfurique et l'acide borique.

15. Kit cosmétique pour la déformation permanente des cheveux comprenant dans un premier compartiment une composition de prétraitement telle que définie dans l'une quelconque des revendications 1 à 6, dans un second compartiment une composition de traitement telle que définie dans l'une quelconque des revendications 1 et 8 à 11, et éventuellement dans un troisième compartiment une composition de post-traitement telle que définie dans l'une quelconque des revendications 12 à 14.
